# EUROPÄISCHE PATENTSCHRIFT

(11) **EP 3 423 514 B1**
(45) Veröffentlichungstag und Bekanntmachung des Hinweises auf die Patenterteilung: **02.09.2020**
(21) Anmeldenummer: 17717059.4
(22) Anmeldetag: 02.03.2017
(51) Int. Cl.: C08J 3/26, C08J 5/02, B29C 41/14, B29C 41/00, A41D 19/00, B29C 41/22

(54) **VERFAHREN ZUM HERSTELLEN EINES PROPHYLAXEARTIKELS**
METHOD FOR PRODUCING A PROPHYLACTIC ARTICLE
PROCÉDÉ DE FABRICATION D'UN ARTICLE PROPHYLACTIQUE

(30) Priorität: 04.03.2016 AT 501752016
(43) Veröffentlichungstag der Anmeldung: 09.01.2019
(73) Patentinhaber: Semperit Aktiengesellschaft Holding, 1031 Wien (AT)
(72) Erfinder: HOLZNER, Armin, 2630 Ternitz (AT); KERN, Wolfgang, 8055 Seiersberg (AT); MANHART, Jakob Cornelius, 2372 Giesshübl (AT); SAHIN, Melahat, 8700 Leoben (AT); SCHALLER, Raimund, 2620 Neunkirchen (AT); SCHLÖGL, Sandra, 8152 Stallhofen (AT)
(74) Vertreter: Burger, Hannes
(86) Internationale Anmeldenummer: PCT/AT2017/060052
(87) Internationale Veröffentlichungsnummer: WO 2017/147638

(56) Entgegenhaltungen:
- EP-A1- 2 389 820
- EP-A1- 2 719 710
- Universität Bayreuth: "Clay and Clay Minerals: Hectorite Festkörperpraktikum Modul AC III 2014", , 1. Januar 2014 (2014-01-01), XP055389066, Gefunden im Internet: URL:http://www.ac1.uni-bayreuth.de/de/teac hing/downloads/Ton_and_Tonminerale_Hectori t_english.pdf [gefunden am 2017-07-07]

## Beschreibung

Die Erfindung betrifft ein Verfahren zum Herstellen eines Prophylaxeartikels, insbesondere eines Handschuhs, aus einem (carboxylierten) Dienkautschuk, nach dem auf eine Form zumindest eine Schicht aus einem (carboxylierten) Dienlatex aufgebracht wird, und der (carboxylierte) Dienlatex mit einem Vernetzungsmittel vernetzt wird.

Weiter betrifft die Erfindung einen Prophylaxeartikel, insbesondere Handschuh, umfassend eine Schicht aus einem (carboxylierten) Dienelastomer, wobei die (carboxylierten) Dienelastomermolekülketten des (carboxylierten) Dienelastomers kovalent über organische Moleküle und ionisch über Metallkationen vernetzt sind.

Prophylaxeartikel, wie insbesondere Operations- und Untersuchungshandschuhe, werden üblicherweise aus einem Elastomerlatex durch eintauchen von handförmigen Tauchformen hergestellt. Auf den Tauchformen bildet sich ein Film, aus dem in weiterer Folge der fertige Einweghandschuh durch Vulkanisation bzw. Vernetzung des Latex entsteht.

Prophylaxeartikel aus Naturlatex weisen ein relativ hohes Allergiepotential auf. Aus diesem Grund werden vermehrt Syntheselatices zur Herstellung der Prophylaxeartikel verwendet. Aber auch diese sind nicht gänzlich hypoallergen, da sie nach wie vor Allergene aus dem Herstellungsprozess aufweisen können, wie beispielsweise Puder zur Verbesserung der Anziehbarkeit, oder Prozesschemikalien, wie beispielsweise Vernetzungschemikalien oder Vernetzungsbeschleuniger.

Um diesen Problemen zu begegnen, wurden im Stand der Technik bereits Verfahren zur Herstellung von Prophylaxeartikel mit reduziertem Allergiepotential vorgeschlagen.

Beispielsweise beschreibt die WO 2011/068394 A1 ein Verfahren, nach dem einem carboxylierten Nitrilbutadien eine Methacrylsäure und ZnO zugesetzt werden. Dadurch erlangt diese Mischung selbstvernetzende Eigenschaften, sodass auf Schwefel enthaltende Vernetzer und Beschleuniger verzichtet werden kann. Nach wie vor enthält diese Zusammensetzung aber das Schwermetall Zn, sodass ein gewisses Allergierestpotential verbleibt.

Ähnlich dazu beschreibt die US 2010/0152365 A1 die Verwendung eines carboxylierten Nitrilbutadien Copolymers zur Herstellung eines Handschuhs mittels Tauchverfahren. Wiederum wird ZnO zur ionischen Vernetzung eingesetzt.

Zur Einstellung bestimmter mechanischer Eigenschaften von Elastomerhandschuhen aus XNBR ist es bekannt, dem Latex Füllstoffe zuzusetzen. Beispielsweise beschreibt H. Mohd. Ghazaly et al, "Some Factors Affecting Dipped Nitrile Latex Films",,,J. Rubb. Res., 4(2), 88-101, den Einsatz von pyrogener Kieselsäure und silanmodifizierter Kieselsäure, wobei in dieser Publikation festgehalten wird, dass keine signifikanten Änderungen der Filmbildungseigenschaften durch die Verwendung von silanmodifizierter Kieselsäure festgestellt werden konnte. Zur Vernetzung wird entweder Schwefel oder ZnO eingesetzt.

Aus Tutchawan Siriyong und Wirunya Keawwattana, "Utilization of Different Curing Systems and Natural Zeolite as Filler and Absorbent for Natural Rubber/Nitrile Rubber Blend", Kasetsart J. (Nat. Sci.) 46 : 918 - 930 (2012), ist bekannt, dass sich durch die Verwendung von Zeolith in NR/XNBR Blends die Zugfestigkeit und das 100 % Modul erhöht. Die Vernetzung erfolgt durch konventionelle Schwefel-Vulkanisation oder mittels peroxidischer Vernetzung. Dabei zeigten die Schwefelsysteme die größte Erhöhung der Zugfestigkeit. Der Zeolith wird als Sorbens eingesetzt, um die Ölresistenz des Gummiprodukts zu erhöhen. Für diese Anwendung erbringt die peroxidische Vernetzung die besten Ergebnisse. In Hinblick auf die Herstellung von Prophylaxeartikel, insbesondere Handschuhe, ist aufgrund deren Dünnwandigkeit basierend auf den Ergebnissen in dieser Publikation, insbesondere der erhöhten Zugfestigkeit, die Schwefelvernetzung zu bevorzugen. Die Erhöhung des 100 % Moduls spricht hingegen gegen die Verwendung dieser Ergebnisse in der Herstellung von Handschuhen, da damit der Tragekomfort reduziert wird.

Es ist weiter bekannt, die Oberfläche von Naturkautschukhandschuhen zu modifizieren, um dessen Allergiepotential zu reduzieren. So beschreibt beispielsweise die von der Anmelderin stammende US 2014/0096307 A1 ein Verfahren zur Modifizierung der Oberfläche eines Elastomers mit ungesättigten Kohlenstoff-Kohlenstoff-Bindungen, die im Bereich der Oberfläche zumindest teilweise durch eine photochemische Reaktion mit zumindest einem Thiol gesättigt werden. Zur Sättigung können Feststoffpartikel, u.a. Zeolithpartikel, verwendet werden, die kovalent an die Oberfläche des Handschuhs gebunden werden. Diese Modifizierung der Handschuhoberfläche erfolgt nach dem Tauchen der Trägerschicht aus dem Naturkautschuk durch Aufbringen der Partikel auf die Oberfläche der Trägerschicht. Im fertigen Handschuh sind die Partikel somit auf der Innenseite der Handschuhe, da diese nach dem Tauchen zum Abziehen gewendet werden.

Ähnlich dazu beschreibt die ebenfalls auf die Anmelderin zurückgehende US 2014/0096308 A1 u.a. die Anbindung von Zeolithpartikel an einen Naturkautschukhandschuh über Epoxidgruppen.

Aus der EP 2 389 820 A1 ist ein Verfahren zum Herstellen eines Handschuhs durch Tauchen einer Handschuhform in ein Tauchbad, in dem ein Latex vorgelegt ist, bekannt, wobei in dem Latex zumindest ein Schichtsilikat als Füllstoff enthalten ist, und der Füllstoff vor der Zugabe zu dem Latex in eine wässrige Dispersion überführt wird.

Die EP 2 719 710 A1 beschreibt ein Verfahren zur Anbindung von Partikeln an die Oberfläche eines Elastomers, insbesondere eines Handschuhs, wobei die Oberfläche des Elastomers zumindest teilweise epoxidiert wird, und wobei die Partikel nach der Epoxidierung der Elastomeroberfläche an die Epoxidgruppen kovalent angebunden werden.

Der vorliegenden Erfindung liegt die Aufgabe zugrunde, einen verbesserten Prophylaxeartikel zu schaffen.

Die Aufgabe wird bei dem eingangs genannten Verfahren dadurch gelöst, dass das Vernetzungsmittel auf anorganischen und/oder organischen Partikeln unter Bildung von modifizierten Partikeln immobilisiert wird und die modifizierten Partikel dem (carboxylierten) Dienlatex zugesetzt werden und dass als Vernetzungsmittel ausschließlich die modifizierten Partikel verwendet werden.

Weiter wird die Aufgabe der Erfindung durch den eingangs genannten Prophylaxeartikel gelöst, bei dem die Metallkationen ausschließlich Teil von anorganischen Partikeln sind, und die organischen Moleküle ausschließlich auf den anorganischen Partikeln immobilisiert sind.

Von Vorteil ist dabei, dass die Partikel nicht oder nur sehr langsam aus dem Prophylaxeartikel migrieren. Mit langsam ist dabei gemeint, dass die Migrationszeit sehr viel größer ist, als die Anwendungszeit des Prophylaxeartikels. Es wird damit verhindert, dass das Vernetzungsmittel in Kontakt mit der menschlichen Haut gelangt, wodurch das Allergiepotential des Prophylaxeartikels deutlich gesenkt werden kann. Auch während der Lagerung der Prophylaxeartikel kann die Migration des Vernetzungsmittels aus dem Prophylaxeartikel verhindert bzw. deutlich verringert werden. Zudem können dadurch Leachingprozesse zur Entfernung nicht gebundener Prozesschemikalien verkürzt oder sogar eingespart werden. Das immobilisierte Vernetzungsmittel kann ein mehrfach funktionelles Monomer und/oder Polymer bzw. Mischungen daraus sein. Mit dem Verfahren kann ein Prophylaxeartikel hergestellt werden, der sehr gute mechanische Eigenschaften und eine hohe Alterungs- und Gammabeständigkeit aufweist. Auch konnte eine Beeinflussung der Filmbildung während des Herstellungsprozesses, insbesondere des Tauchprozesses, nicht nachgewiesen werden, sodass also keine weiteren Maßnahmen hierfür erforderlich sind. Ein weiterer Vorteil des Verfahrens ist darin zu sehen, dass eine Vorvernetzung des (carboxylierten) Dienlatex nicht erforderlich ist, sodass kontinuierliche Mischverfahren eingesetzt und die Prozessabläufe beschleunigt werden können. Es ist mit dem Verfahren eine energieeffiziente, nachhaltige und produktionseffiziente Herstellung von hypoallergenen Prophylaxeartikeln, insbesondere Operations- und Untersuchungshandschuhen, möglich.

Als Vernetzungsmittel werden ausschließlich die modifizierten Partikel verwendet. Es können damit die voranstehend genannten Effekte weiter verbessert werden, wobei zusätzlich noch erreicht werden kann, dass durch den Verzicht von Schwermetallionen, wie beispielsweise Zn²⁺ aus ZnO, das Allergiepotential weiter verringert werden kann (Zink kann beispielsweise mit Carbonsäuren, wie z.B. Essigsäure, aus dem Elastomer extrahiert werden). Darüber hinaus kann keine Beeinflussung eines anderen Vernetzersystems stattfinden, wie dies teilweise im Stand der Technik berichtet wird.

Nach einer bevorzugten Ausführungsvariante des Verfahrens kann vorgesehen sein, dass als anorganische Partikeln silikatbasierende Partikel eingesetzt werden. Mit silikatbasierenden Partikeln ist über deren Kation, beispielsweise Ca²⁺, neben der kovalenten Vernetzung der Moleküle des (carboxylierten) Dienlatex zusätzlich eine ionische Vernetzung erzielbar. Es ist damit einfacher möglich, zur Erreichung höherer Reißfestigkeiten des Prophylaxeartikels auf zusätzliche Vernetzungsmittel, beispielsweise das häufig verwendete ZnO, zu verzichten. Höhere Reißfestigkeiten werden über die ionischen Netzwerkstellen erreicht, während kovalente Bindungen eine Verbesserung in Hinblick auf die Reduktion der Lochbildungsanfälligkeit des Prophylaxeartikels beim Tragen bewirken. Nachdem die Partikel insbesondere physikalisch und/oder mechanisch gebunden in das Elastomer eingebettet werden, wandern diese auch nicht aus dem Prophylaxeartikel aus, sodass auch keine Probleme mit Pulvern in Hinblick auf das Allergiepotential des Prophylaxeartikels entstehen.

Nach einer anderen Ausführungsvariante des Verfahrens ist vorgesehen, dass die silikatbasierenden Partikel ausgewählt werden aus einer Gruppe bestehend aus Silikaten mit mehrwertigen Kationen, Zeolithen, SiO₂ sowie Mischungen daraus. Es ist damit eine weitere Verbesserung der voranstehend genannten Effekte erreichbar. Zudem kann mit Zeolith eine bessere Einbindung der silikatbasierenden Partikel in die Elastomerschicht über dessen Hohlräume erreicht werden, sodass also die silikatbasierenden Partikel einfacher ausschließlich physikalisch oder mechanisch mit der ersten Schicht verbunden werden können. Zudem kann damit der Zusatznutzen erreicht werden, dass diese Partikel auch adsorbierend für allfällige in die erste Schicht von außen oder aus der Innenseite des Prophylaxeartikels einwandernde Schadstoffe wirken können.

Gemäß einer Ausführungsvariante dazu kann vorgesehen sein, dass der Zeolith ein natürlicher Zeolith ist. Neben dem Kostenfaktor - synthetische Zeolithe sind deutlich teurer als natürliche - ist hierbei von Vorteil, dass natürliche Zeolithe im Vergleich zu synthetischen eine uneinheitlichere Struktur aufweisen. Damit werden durch den natürlichen Zeolith an sich die mechanischen Eigenschaften des Prophylaxeartikels in Summe weniger beeinflusst, als durch den Einsatz eines synthetischen Zeoliths.

Als besonders geeignet hat sich im Zuge von durchgeführten Tests ein Zeolith herausgestellt, der ausgewählt ist aus einer Gruppe bestehend aus Klinoptilolith, Chabasit, Phillipsit, Analcim sowie Mischungen hiervon. Dies ist umso überraschender, als diese Zeolithe zu unterschiedlichen Strukturklassen gehören.

Von Vorteil ist auch, wenn die Partikel mit einem Überschuss an Vernetzungsmittel, insbesondere mit Siloxanen mit Epoxygruppen, zur Ausbildung eine Mehrschichtstruktur des Vernetzungsmittels auf den Partikeln modifiziert werden. Durch die kovalente Anbindung des Vernetzungsmittels an die Partikel, wird ein Teil der Ankergruppen/netzwerkbildenden Gruppen hydrolysiert. Diese stehen somit für die Vernetzung nicht mehr zur Verfügung. Aus diesem Grund ist die Verwendung eines Überschusses an Vernetzungsmittel von Vorteil, da damit eine Mehrschichtstruktur auf der Oberfläche der Partikel ausgebildet wird. In dieser Mehrschichtstruktur ist die erste Schicht nach wie vor kovalent an die Partikel gebunden und hydrolysiert, in den weiteren Schichten erfolgt aber keine Hydrolyse der Ankergruppen/netzwerkbildenden Gruppen, sodass insgesamt die Reaktivität der mit dem Vernetzungsmittel versehenen Partikel besser ist.

Die Vernetzung der (carboxylierten) Dienlatexmoleküle kann thermisch durchgeführt werden. Somit kann die Vernetzung der Latexmoleküle bereits während des Trocknens des auf die Tauchform aufgetauchten Latexfilms erfolgen, wodurch eine Effizienzsteigerung des Verfahrens erzielbar ist. Insbesondere ist die Ausführungsvariante von Vorteil, wenn die Partikel mit einem Überschuss an Vernetzungsmittel versetzt werden.

Vorzugsweise wird der pH-Wert des (carboxylierten) Dienlatex auf einen Wert von größer/gleich 9 eingestellt. Es konnte mit pH-Werten ab 9 eine deutliche Verbesserung der Reaktionskinetik beobachtet werden, wodurch die Vernetzung der Moleküle rascher erfolgen kann.

Das Vernetzungsmittel kann ausgewählt werden aus einer Gruppe bestehend aus mehrfachfunktionellen Epoxiden, mehrfachfunktionellen Silanen, mehrfachfunktionellen Siloxanen, mehrfachfunktionellen Thiolen. Von Vorteil ist dabei, wenn diese (i) wasserlöslich sind, da bei der Einbringung des Vernetzungsmittels in die Latexmischung kein Emulgator benötigt wird; (ii) mehr als eine Epoxidfunktion für die Vernetzung der Kautschukketten haben. Vorzugsweise wiesen die mehrfachfunktionellen Epoxide eine Struktur auf, dass das Hydrolyseprodukt "pflegende" Eigenschaften aufweist, wie beispielsweise diglycidylterminiertes Polyethylenglykolderivat, Epoxy-Sorbitolderivat, Derivat eines Zuckeralkohols. Weiters können beispielsweise. Mono- und Polysaccharide mit Epoxy-Funktionalitäten eingesetzt werden.

Von Vorteil bei den mehrfachfunktionellen Thiolen ist, wenn diese (i) eine hohe Molmasse besitzen (Molmasse zwischen 200 g/mol und 4000 g/mol); (ii) eine hohe Mercaptoequivalentzahl aufweisen (zumindest 20%, insbesondere zumindest 50 %, der Monomereinheiten sollten SH-Gruppen tragen); (iii) über einfache Synthesestrategien zugänglich sind.

Von Vorteil bei den mehrfachfunktionellen Silane und Siloxane ist, wenn diese (i) mehr als eine reaktive Gruppe tragen (z.B. CoatOSil MP200 führt zu höheren Reißfestigkeiten als 3-Glycidoxypropyltrimethoxysilan); (ii) die Silane zumindest eine Trialkoxygruppe tragen (zur Ankopplung an den Füllstoff und zur Ausbildung von Oligolagen über physikalische Wechselwirkungen).

Zum besseren Verständnis der Erfindung wird diese anhand der nachfolgenden Figuren näher erläutert.

Es zeigen:
- Fig. 1: den Quellgrad von vernetzten XNBR-Latexfilmen (nicht vorvernetzt; thermische Vernetzung im Zuge der Trocknung bei 100°C für 15min) bei unterschiedlichen Rima Sil 1200-Konzentrationen;
- Fig. 2: Darstellung der kovalenten (links) und der ionischen (rechts) Vernetzung von XNBR;
- Fig. 3: Reißfestigkeiten von vernetzten XNBR-Latexfilmen bei unterschiedlichen Rima Sil 1200-Konzentrationen (keine Vorvernetzung; thermische Vernetzung im Zuge der Trocknung bei 100°C für 15min);
- Fig. 4: Zugfestigkeiten von XNBR-Latexfilmen bei unterschiedlichen Prävulkanisationszeiten und bei Einsatz von 5phr Rima Sil 1200;
- Fig. 5: Bruchdehnungen von vernetzten XNBR-Latexfilmen bei unterschiedlichen Rima Sil 1200-Konzentrationen (keine Vorvernetzung; thermische Vernetzung im Zuge der Trocknung bei 100°C für 15min);
- Fig. 6: Spannungswert bei 50% Dehnung von vernetzten XNBR-Latexfilmen bei unterschiedlichen Rima Sil 1200-Konzentrationen (keine Vorvernetzung; thermische Vernetzung im Zuge der Trocknung bei 100°C für 15min);
- Fig. 7: Reißfestigkeiten von XNBR-Latexfilmen bei unterschiedlichen Partikeltypen und Partikelkonzentrationen;
- Fig. 8: Bruchdehnungen von XNBR-Latexfilmen bei unterschiedlichen Partikeltypen und Partikelkonzentrationen;
- Fig. 9: Spannungswerte bei 50% Dehnung von XNBR-Latexfilmen bei unterschiedlichen Partikeltypen und Partikelkonzentrationen;
- Fig. 10: Reißfestigkeiten von XNBR-Latexfilmen bei unterschiedlichen Partikeltypen und Partikelkonzentrationen (Vernetzerkonzentration: 5phr).

Sämtliche in der Beschreibung zitierten Normen beziehen sich auf die zum Anmeldezeitpunkt gegenständlicher Patentanmeldung gültige Fassung, sofern nichts anderes angegeben ist.

Die Erfindung bezieht sich auf ein Verfahren zum Herstellen eines Prophylaxeartikels.

Der Prophylaxeartikel ist bevorzugt ein Handschuh, insbesondere ein chirurgischer Handschuh (Operationshandschuh) oder ein Untersuchungshandschuh. Der Prophylaxeartikel kann aber beispielsweise auch ein Fingerling, ein Katheter, ein Kondom, ein (medizinischer) Ballon, ein Sauger, etc., sein. Generell ist der Prophylaxeartikel bevorzugt ein Tauchartikel, also ein Produkt, das mittels eines Tauchverfahrens hergestellt wird.

Im Folgenden wird nur mehr auf die Ausbildung des Prophylaxeartikels als Handschuh eingegangen. Die Ausführungen dazu können aber auch auf andere Elastomerartikel, insbesondere Tauchartikel die nach einem Tauchverfahren hergestellt werden, übertragen werden.

Der Handschuh umfasst ein Dienelastomer (Dienkautschuk), insbesondere ein carboxyliertes Dienelastomer, bzw. besteht aus diesem.

Das Elastomer der Elastomerschicht kann sowohl auf einem Natur- als auch auf einem Syntheselatex basieren. Diese können ausgewählt sein aus einer Gruppe umfassend oder bestehend aus Naturkautschuk (NR), Polyisopren-Latex (IR), Nitrilbutadienkautschuk-Latex (NBR), carboxylierter Nitrilbutadienkautschuk-Latex (XNBR), carboxylierter Butadienlatex (XBR), Chloropren-Latex (CR), Styrol-Butadien Latex (SBR), carboxylierte Latices hergestellt aus Polymer-Blends, sowie Mischungen daraus.

Insbesondere wird ein carboxylierter Nitrilbutadienkautschuk-Latex zur Herstellung der Elastomerschicht verwendet. Dieser weist bevorzugt einen Anteil an Acrylnitril zwischen 15 Gew.-% und 40 Gew.-%, insbesondere zwischen 20 Gew.-% und 35 Gew.-%, auf.

Der Prophylaxeartikel bzw. der Elastomerhandschuh wird bevorzugt nach einem Tauchverfahren hergestellt. Derartige Tauchverfahren sind aus dem Stand der Technik prinzipiell bekannt, sodass zu Einzelheiten dazu auf den einschlägigen Stand der Technik verwiesen sei.

Im Wesentlichen wird bei diesem Verfahren eine Tauchform (üblicherweise werden in der seriellen Fertigung mehrere Tauchformen verwendet) in ein Tauchbad eingetaucht. Die Tauchform weist dabei die Form des fertigen Produktes auf, also beispielsweise die Form einer Hand.

Im Tauchbad ist der jeweilige Elastomerlatex vorgelegt, der auf die Tauchform aufgetaucht werden soll.

Prinzipiell kann aber auch jede andere geeignete Form in den in dieser Beschreibung beschriebenen Verfahren verwendet werden, insbesondere wenn die Elastomerschicht nicht nach einem Tauchverfahren hergestellt wird. Die Elastomerschicht kann beispielsweise auch durch aufstreichen oder aufsprühen des Elastomerlatex auf eine Form hergestellt werden. Ebenso sind auch andere geeignete Verfahren der Aufbringung des Latex auf eine Form anwendbar.

Der Begriff Elastomerlatex wird in dieser Beschreibung dem üblichen Gebrauch in der Fachsprache entsprechend verwendet. Dementsprechend ist ein Elastomerlatex eine Dispersion aus unvernetzten oder vorvernetzten oder vernetzungsfähigen Polymermolekülen zur Herstellung eines Elastomers. Es können im Rahmen der Erfindung also auch vorvernetzte Elastomerlatices verarbeitet werden, wobei die Vorvernetzung insbesondere mittels den in dieser Beschreibung genannten Vernetzungsmitteln erfolgen kann.

Es ist aber weiter möglich, dass der Elastomerlatex erst nach dem Aufbringen auf die Form, also der aufgebrachte Elastomerlatex, vernetzt wird.

Eine übliche Prozessroute eines Koagulations-Tauchverfahrens kann beispielsweise folgende Verfahrensschritte umfassen:
- waschen der Tauchform und entfetten mit einem organischen Lösungsmittel;
- vorwärmen der Tauchform;
- tauchen der Tauchform in ein erstes Tauchbad mit einem Koagulanten;
- Antrocknen der ersten aufgetauchten Schicht;
- tauchen der Tauchform in ein weiteres Tauchbad zur Ausbildung der Elastomerschicht;
- trocknen/vulkanisieren (vernetzen);
- abziehen des Tauchartikels von der Form.

Für den Fall, dass der Elastomerhandschuh mehrschichtig ausgebildet wird, können weitere Schichten aus dem ersten Elastomerlatex oder aus einem anderen Elastomerlatex oder einem anderen Polymer aufgetaucht oder generell aufgebracht werden. Beispielsweise kann als letzte Schicht eine Polymerschicht aufgetaucht werden, die nach dem Abziehen des Handschuhs von der Tauchform durch das dabei erfolgende Wenden des Handschuhs an die Innenseite des Handschuhs gelangt. Derartige Polymerschichten können beispielsweise als Gleitschichten ausgebildet sein, um die Anziehbarkeit des Elastomerhandschuhs zu verbessern.

Der Elastomerhandschuh kann also ein- oder mehrschichtig ausgebildet sein, wobei die einzelnen Schichten aus zueinander unterschiedlichen Werkstoffen oder aus den gleichen Werkstoffen bestehen können. Es ist auch möglich, dass zwei oder mehr Schichten des Elastomerhandschuhs aus dem gleichen Werkstoff und eine oder mehr Schichten aus einem dazu unterschiedlichen Werkstoff bestehen.

Da all dies an sich bekannt ist, soll nicht weiter darauf eingegangen werden.

Unter Werkstoffen werden dabei in dieser Beschreibung Elastomere und Polymere verstanden, wobei aber der Elastomerhandschuh zumindest eine Schicht aus einem Elastomer aufweist.

Die Begriffe Vulkanisation und Vernetzung werden in dieser Beschreibung synonym verwendet.

Zur Vernetzung des (carboxylierten) Dienelastomer-Latex wird diesem, d.h. insbesondere dem Tauchbad zur Herstellung der zumindest einen Schicht aus dem (carboxylierten) Dienelastomers, ein Vernetzungsmittel zugesetzt. Daneben kann der Dienelastomer-Latex bzw. das Tauchbad zumindest ein weiteres Additiv, wie beispielsweise zumindest einen Emulgator, zumindest ein Alterungsschutzmittel, zumindest einen Farbstoff, zumindest ein Antiozonat, aufweisen, wie sie an sich für die Herstellung von Tauchartikeln bekannt sind. Der Gesamtanteil an diesen Additiven kann zwischen 0,1 phr und 10 phr, bezogen auf die Gesamtzusammensetzung des Dienelastomer-Latex bzw. des Tauchbads, betragen.

Das Vernetzungsmittel wird dem (carboxylierten) Dienelastomer-Latex auf anorganischen und/oder organischen Partikeln unter Bildung von modifizierten Partikeln immobilisiert zugesetzt. Die Partikel sind in dem (carboxylierten) Dienelastomer-Latex nicht löslich. Es besteht auch die Möglichkeit, dass die Partikel mit einem mehrfach funktionellen Monomer und/oder Polymer, das an sich im (carboxylierten) Dienelastomer-Latex aufgelöst oder emulgiert werden kann und das als Vernetzungsmittel dient, modifiziert werden.

Als Vernetzungsmittel werden ausschließlich die modifizierten Partikel verwendet.

Es ist weiter bevorzugt, wenn der (carboxylierte) Dienelastomer-Latex nach dem Aufbringen auf die Form vernetzt wird. Es ist aber auch eine Vorvernetzung des (carboxylierten) Dienelastomer-Latex möglich, beispielsweise auch mit den Partikeln, auf denen das Vernetzungsmittel immobilisiert ist.

Die verwendeten Partikel können anorganischer und/oder organischer Natur sein.

Die organischen Partikel können ausgewählt sein aus einer Gruppe umfassend oder bestehend aus Polydimethylsiloxanen, Silikonharzen, Harnstoffharzen, Epoxidharzen, Dien-Elastomeren.

Die anorganischen Partikel können ausgewählt sein aus einer Gruppe bestehend aus oder umfassend silikatbasierende Partikel, SiO₂, Carbonate, Oxide.

Bevorzugt werden als anorganische Partikel silikatbasierende Partikel verwendet.

Die silikatbasierenden Partikel werden vorzugsweise ausgewählt aus einer Gruppe umfassend oder bestehend aus Silikaten mit mehrwertigen Kationen, beispielsweise Wollastonit, Zeolithen, sowie Mischungen daraus.

Nach einer Ausführungsvariante des Elastomerhandschuhs und dessen Verfahren zu Herstellung bestehen die silikatbasierenden Partikel aus einem Zeolith, insbesondere einem natürlichen Zeolith, wobei bevorzugt ist, als Zeolith Klinoptilolith, Chabasit, Phillipsit, Analcim sowie Mischungen hiervon einzusetzen. Gemäß einer weiteren bevorzugten Ausführungsvariante wird als natürlicher Zeolith ein Klinoptilolith eingesetzt. Es ist jedoch auch möglich, dass der Zeolith ein synthetischer Zeolith ist.

Natürlicher Zeolith weist je nach Lagerstätte einen mehr oder weniger großen Anteil an Begleitmineralien, insbesondere Quarz, auf. Für den Einsatz von natürlichen Zeolith in dem Verfahren zur Herstellung des Elastomerhandschuhs wird vorzugsweise ein natürlicher Zeolith verwendet, der einen Reinheitsgrad von mindestens 85 %, insbesondere mindestens 90 %, aufweist, d.h. dass zumindest 80 % bzw. 90 % der silikatbasierenden Partikel aus dem Zeolith bestehen.

Die Partikel werden dem (carboxylierten) Dienelastomer-Latex zugesetzt. Dadurch werden diese Partikel in die Schicht aus dem Elastomerlatex eingebunden bzw. eingebettet, sodass also die Partikel physikalisch und/oder mechanisch eingebunden werden. Oberflächennahe Partikel (in Bezug auf die Schicht aus dem (carboxylierten) Dienelastomer) können über die Elastomerschicht vorragen, wobei in diesem Fall zumindest zu 90 %; insbesondere 100 %, dieser Partikel ebenfalls mit dem Elastomer der Elastomerschicht überzogen sind. Es kann damit eine Oberflächenrauheit erzeugt werden, die die Griffigkeit des Handschuhs verbessern kann bzw. die die Verbundfestigkeit der Elastomerschicht mit einer weiteren Schicht verbessern kann, indem zusätzlich eine mechanische Verankerung der Partikel in der weiteren Schicht erreicht werden kann.

Das Vorragen der Partikel über die Oberfläche der Elastomerschicht kann durch die Partikelgröße der Partikel und/oder die Schichtdicke der Elastomerschicht erreicht bzw. eingestellt werden.

Die Partikel können eine Partikelgröße aufweisen, die einer Partikelgrößenverteilung mit einem mittleren Partikeldurchmesser (d₅₀) von 0,5 µm bis 7,5 µm bei einem Top Cut (d₉₈) von 2 µm bis 20 µm entsprechen.

Die Größen der natürlichen Zeolithpartikel wurden mit einem Malvern Mastersizer, Hydro 2000G (Nasszelle) bestimmt. Die Partikelgröße der synthetischen Zeolithe sowie der Silikatpartikel wurden aus dem Datenblatt der Hersteller entnommen.

Eingesetzte Partikel für die nachfolgenden Beispiele:
Zeolith 1: natürlicher Zeolith; d₁₀= 2 µm; d₅₀= 5 µm; d₉₈= 15 µm (BET: 33,2 m²/g).
Zeolith 2: natürlicher Zeolith; d₁₀= 1,7 µm; d₅₀= 3 µm; d₉₈= 8,5 µm.
Zeolith 3: natürlicher Zeolith; d₁₀= 0,3 µm; d₅₀= 1,4 µm; d₉₈= 5,5 µm.
Zeolith 4: synthetischer Zeolith; stäbchenförmig: 300x700nm (BET: 300 m²/g).
Silika 1: Amorphe Kieselsäure (Sigma-Aldrich): 0.2-0.3 µm (Aggregate) (BET: 200 m²/g).
Silika 2: Kieselsäure mit der Bezeichnung KS 400 von der Firma Grace, BET = 180 m²/g.
Rima Sil 1200: Ca-Silikat (CaSiO₃), d₅₀=2µm.

Die Schichtdicke der Elastomerschicht kann zwischen 30 µm und 500 µm betragen.

Prinzipiell können die Partikel jeden Habitus aufweisen. Bevorzugt werden jedoch Partikel verwendet, die zumindest annähernd rund bzw. verrundet sind, also keine scharfen Bruchkanten aufweisen.

Vorzugsweise sind die Partikel in einem Massenanteil von 1 phr (parts per hundred rubber) bis 20 phr, insbesondere von 3 phr bis 10 phr, in der Elastomerschicht enthalten.

Gemäß einer weiteren Ausführungsvariante können die Partikel vorzugsweise eine spezifische BET Oberfläche zwischen 1 g/m² und 300 g/m² aufweisen. Es können damit die mechanischen Eigenschaften aufgrund der Wechselwirkungen der Partikel mit der Elastomermatrix verbessert werden. Zudem können damit auf der Oberfläche der Partikel mehr kovalente Bindungen entstehen.

Zur Modifikation der Partikel können Vernetzungsmittel verwendet werden, die ausgewählt sind aus einer Gruppe bestehend aus oder umfassend (mehrfachfunktionelle) Epoxide, (mehrfachfunktionelle) Silane, Dialkoxysilane, Trialkoxysilane, Trichlorsilane. Beispiele dafür sind (3-Glycidoxypropyl) trimethoxysilan, Polyglycidoxypropyltrimethoxysilan, (3-Glycidoxypropyl) triethoxysilan, (3-Glycidoxypropyl) trichlorsilan, 2-(3,4-Epoxycyclohexyl)ethyltriethoxysilan, 2-(3,4-Epoxycyclohexyl)ethyltrimethoxysilan, 5,6-Epoxyhxyltriethoxysilan, (3 -Glycidoxypropyl)methyldiethoxysilan, (3-Glycidoxypropyl)methyldimethoxysilan.

Die Partikel werden in der Regel oberflächlich modifiziert. Werden jedoch Partikel mit Hohlräumen eingesetzt, wie beispielsweise Zeolithe, kann die Modifikation auch die Oberfläche der Hohlräume umfassen.

Die Partikel werden vorzugsweise mit einem Überschuss an Vernetzungsmittel zur Ausbildung eine Mehrschichtstruktur des Vernetzungsmittels auf den Partikeln modifiziert. Es ist jedoch auch möglich, eine dazu geringere Menge an Vernetzungsmittel zu verwenden, sodass das Vernetzungsmittel im Unterschuss zur zu modifizierenden Oberfläche, d.h. den zu modifizierenden reaktiven Gruppen an der Oberfläche der Partikel, vorhanden ist.

Die Modifikation der Partikel kann wie nachstehend ausgeführt erfolgen. Es können aber auch bereits modifizierte Partikel eingesetzt werden. Erhältlich sind derartige Partikel beispielsweise Grolman (Rima Sil 1200 auf Ca-Silikatbasis), EM Hoffmann Minerals (Aktisil EM), Quarzwerke (Tremin 283-400EST).

Wie bereits voranstehend ausgeführt, können die Partikel mit einem Vernetzungsmittel in Form von im (carboxyliertem) Dienelastomer-Latex an sich löslichen oder dispergierbaren bzw. emulgierbaren mehrfachfunktionellen Monomeren und/oder mehrfachfunktionellen Polymeren modifiziert werden. Der Begriff "Polymer" im Sinne dieser Beschreibung umfasst dabei generell Moleküle ab zwei Monomereinheiten, also Moleküle ab Dimeren. Die mehrfachfunktionellen Monomere und/oder Polymere werden vorzugsweise ausgewählt aus einer Gruppe umfassend oder bestehend aus mehrfachfunktionelle(n) Epoxide(n), mehrfachfunktionelle(n) Silane(n), mehrfachfunktionelle(n) Siloxane(n), mehrfachfunktionelle(n) Thiole(n), sowie Mischungen daraus.

Beispiele dafür sind kurzkettige: Sorbitol polyglycidyl ether, Glycerol glycidyl ether, 1,6-Hexandioldiglycidyl ether, Resorcinol diglycidyl ether, 1,4-Cyclohexandimethanol diglycidyl ether, Diglycidyl 1,2-cyclohexandicarboxylat, Ethyleneglycoldiglycidylether langkettige: Diepoxy-terminiertes Polyethylenglykol, Diepoxy-terminiertes Polypropylenglykol, Polyglycidylmethacrylat (Homopolymere und Copolymere mit Ethylenglykoleinheiten, Ethyleneinheiten, etc.), Polyglycerinpolyglycidether.

Kurzkettige Verbindungen sind monomere, hochmolekulare, mehrfachfunktionelle Verbindungen, insbesondere derartige Verbindungen mit einem Molekulargewicht von zumindest 170 g/mol. Langkettige Verbindungen weisen zumindest zwei oder mehrere Wiederholungseinheiten auf (Dimere und größer).

Generell umfasst im Rahmen der Erfindung der Begriff "Polymer" auch Oligomere.

Generell beschreibt der Begriff "hochmolekulares Monomer" ein Monomer, dass ein Molekulargewicht von vorzugsweise zumindest 170 g/mol, insbesondere zwischen 170 g/mol und 4000 g/mol, aufweist.

Es ist weiter von Vorteil, wenn der pH-Wert des (carboxylierten) Dienelastomer-Latex auf einen Wert von größer/gleich 9 eingestellt wird. Dazu kann beispielsweise eine wässerige KOH-Lösung (1Gew.-% bis 5 Gew.-%) verwendet werden. Generell können dazu geeignete basische Substanzen, wie Laugen, verwendet werden.

In der bevorzugten Ausführungsvariante des Verfahrens erfolgt die Vernetzung der (carboxylierten) Dienelastomermoleküle thermisch, insbesondere während der Trocknung der (aufgetauchten) Schicht aus dem (carboxylierten) Dienelastomer-Latex. Die Temperatur kann dabei zwischen 90 °C und 140 °C betragen. Die Vernetzung kann während einer Zeitspanne zwischen 5 Minuten und 20 Minuten erfolgen.

Es kann ein Vernetzungsmittel eingesetzt werden, das ein Molekulargewicht zwischen 170 g/mol und 4000 g/mol, insbesondere zwischen 170 g/mol und 1700 g/mol (polymere, wasserlösliche Verbindungen gemäß DIN 55672-3:2007-08 (GPC)) oder über die Viskosität von flüssigen Polymeren gemäß DIN 51 562-1) aufweist. Beispielsweise kann Ethylenglykoldiglycidylether (Molekulargewicht 170 g/mol) oder Diethylenglykoldiglycidylether (Molekulargewicht 218 g/mol) verwendet werden. Es ist damit auch möglich, das (50%-)Modul des Elastomerhandschuhs auf einen gewünschten Wert einzustellen. Das Modul des Elastomerhandschuhs kann über die Kettenlänge des Vernetzungsmittels eingestellt werden.

Mit dem Verfahren kann ein Prophylaxeartikel, insbesondere Handschuh, hergestellt werden, umfassend eine Schicht aus einem (carboxylierten) Dienelastomer, wobei die (carboxylierten) Dienelastomermolekülketten des (carboxylierten) Dienelastomers kovalent über organische Moleküle und gegebenenfalls ionisch über Metallkationen vernetzt sind, wobei die Metallkationen ausschließlich Teil von anorganischen Partikeln sind, und wobei die organischen Moleküle ausschließlich auf den anorganischen Partikeln immobilisiert sind.

Die nach dem Verfahren hergestellten Elastomerhandschuhe weisen eine gute Hautverträglichkeit auf. Anhand von durchgeführten Untersuchungen konnte kein Hautreizungs- und kein Sensibilisierungspotential festgestellt werden.

Im Zuge der Erprobung des Vernetzungsverfahrens wurden u.a. folgende Experimente durchgeführt. Bei diesen handelt es sich nur um ausgewählte Beispiele, da die Wiedergabe sämtlicher Experimente den Rahmen dieser Beschreibung sprengen würde.

In der folgenden Tabelle 1 sind die in den Experimenten zur Verwendung von modifizierten Partikel (Füllstoffen) verwendeten Materialien zusammengefasst.

**Tabelle 1: verwendete Materialien zur Vernetzung mit modifizierten Partikeln**

| *Name* | *Funktion* | *Beschreibung* |
|---|---|---|
| Nipol LX556 ZEON Corporation (JPN) | Latex | 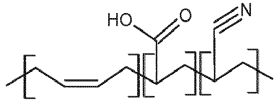 |
| | | XNBR |
| | | Trockenkautschukgehalt: 45,2% |
| | | pH-Wert: 8 bis 8.8 |
| (3-Glycidoxypropyl) trimethoxysilan Wacker | Einfach funktionelles Epoxid zur Partikelmodifikation | 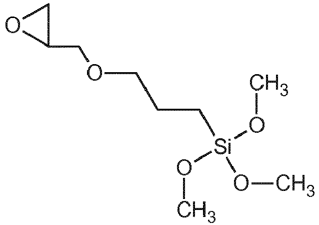 |
| CoatOSil MP 200 Momentive | Dreifach funktionelles Epoxid zur Partikelmodifikation | 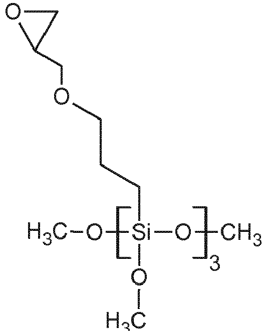 |
| Rima Sil 1200 Grolman Group (GER) | Vernetzer | 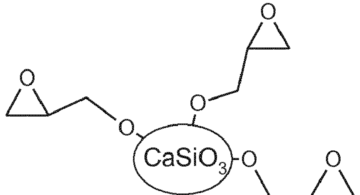 |
| | | Dreifach epoxy-funktionelles Silan auf Calciumsilikat-Träger (∼50% Silananteil an der Oberfläche) |
| Zeolith Inzeo mono 15 5 Paltentaler Minerals | Vernetzer | 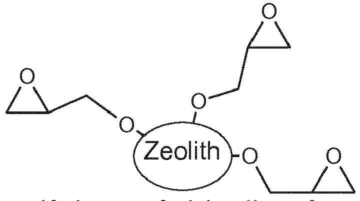 |
| | | Dreifach epoxy-funktionelles auf Zeolith-Träger (∼50% Silananteil an der Oberfläche) |
| Zeolith Inzeo mono 15 5 Paltentaler Minerals | Vernetzer | 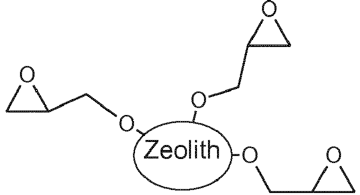 |
| | | Einfach epoxy-funktionelles Silan auf Zeolith-Träger (∼50%) Silananteil an der Oberfläche) |

Die epoxy-funktionalisierten Partikel wurden in unterschiedlicher Konzentration (3 bis 7,5 phr) in deionisiertem Wasser mit einem Ultraturrax (10min bei Raumtemperatur) vordispergiert und im Anschluss der Latexmischung (pH = 10, ∼25 drc. (dry rubber content)) zugegeben.

Generell kann der (carboxylierte) Dienelastomer-Latex einen Feststoffgehalt an (carboxyliertem) Dienelastomer zwischen 10 drc (dry rubber content) und 60 drc aufweisen.

Die Mischung wurde mit einem Alterungsschutzmittel (0,5 phr Ralox) versetzt und etwa 15min bei Raumtemperatur gerührt. Anschließend wurden die Filme mittels dem voranstehend angeführten Koagulationstauchverfahren hergestellt. Dabei wurde die Latexmischung während des Tauchprozesses leicht mit Hilfe eines Magnetrührers gerührt, um eine Sedimentation der Partikel zu unterbinden.

Das Rühren der Latexmischung wird vorzugsweise generell bei dem Verfahren angewandt.

Die Filme wurden bei 100°C für 15min getrocknet. Es wurde keine Vorvernetzung bzw. Latexreifung benötigt, da die Vernetzung während der Trocknung der Filme bei 100°C stattfand.

Nachfolgende Reaktion liegt der thermischen Vernetzung mit epoxid-funktionellen anorganischen Partikeln als Vernetzungsmittel zu Grunde. Bevorzugt wird im Vorfeld die Einstellung des pH-Werts der Latexmischung mit 1 Gew.-%iger KOH auf pH=10 bis 10,5, da die Reaktion bei höheren pH-Werten katalysiert wird.

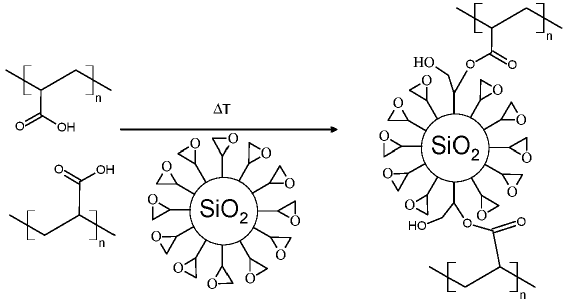

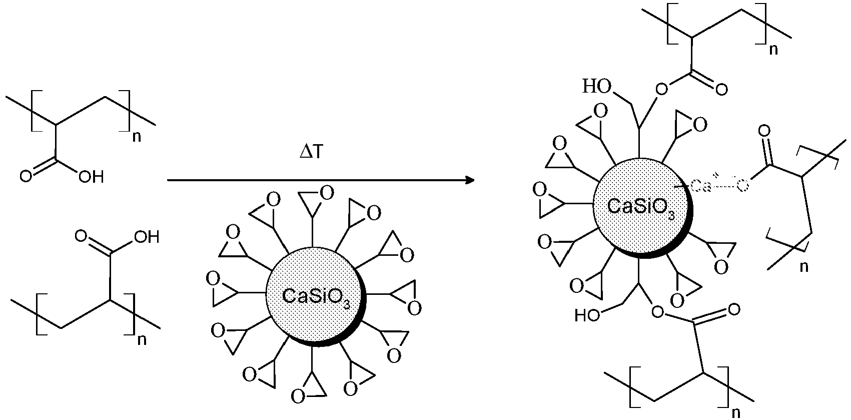

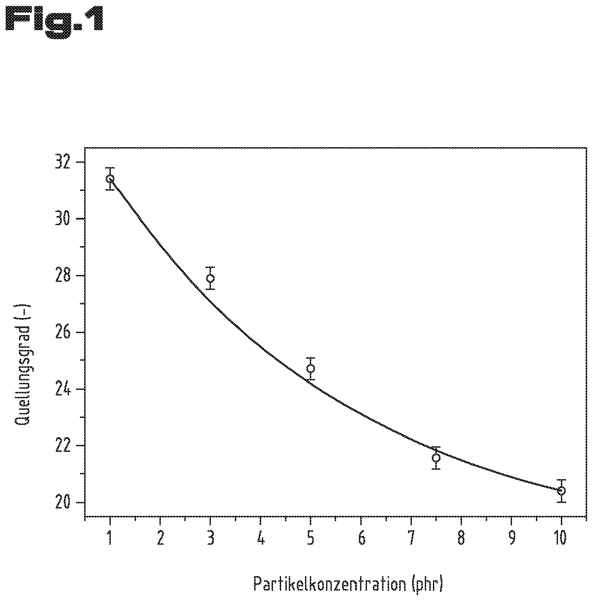

Die erfolgreiche Vernetzung von XNBR-Latex bei Einsatz von funktionellen anorganischen Partikeln als nicht extrahierbare Vernetzungsmittel wurde im ersten Schritt mittels Gleichgewichtsquellungen in Chloroform (bestimmt nach: (1) Macromolecules 2008, 41, 4717-4729, (2) J. Appl. Polym. Sci. 129(5), 2735-2743 bzw. (3) Zaborski, M.; Kosmalska, A.; Gulinski, J. Kautsch. Gummi Kunstst. 2005, 58, 354) nachgewiesen. Die Ergebnisse sind in Fig. 1 dargestellt (Abszisse: Konzentration an Vernetzungsmittel in phr; Ordinate: Quellgrad) und zeigen, dass der Vernetzungsgrad mit der Konzentration des Vernetzerungsmittels korreliert und höhere Vernetzerkonzentrationen zu höheren Vernetzungsdichten führen.

Es wird daher vorzugsweise eine Konzentration an Vernetzungsmittel zwischen 1 phr und 15 phr, insbesondere zwischen 1 phr und 7,5 phr, eingesetzt.

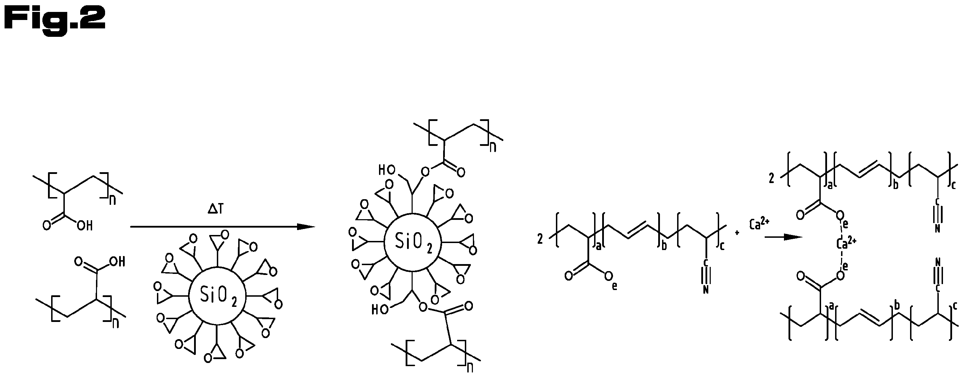

Neben der geringen Extrahierbarkeit, beruht der Vorteil der epoxy-funktionellen Partikel auf Basis von Ca-Silikaten, dass man mit einem Vernetzungsmittel kovalente sowie ionische Vernetzungsstellen ausbilden kann, wie dies in Fig. 2 dargestellt ist (links kovalente Bindungen, rechts ionische Bindungen). Durch diesen kombinierten Ansatz besteht auch die Möglichkeit schwermetallfreie (frei von ZnO) Handschuhe herzustellen.

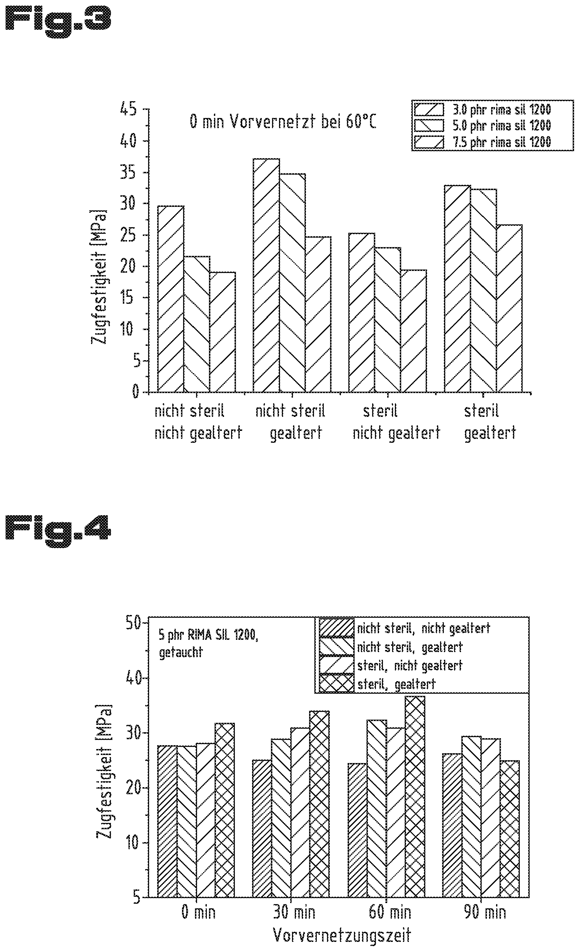

Fig. 3 zeigt die Reißfestigkeit (Ordinate in MPa) in Abhängigkeit von der eingesetzten Partikelmenge. Auf der Abszisse sind dabei die Balken jeweils in Dreiergruppen für 3 phr, 5 phr und 7,5 phr Partikel von links nach rechts für nicht sterile und nicht gealterte, nicht sterile und gealterte, sterile und nicht gealterte sowie sterile und gealterte Proben dargestellt.

Generell kann die Sterilisation durch Gamma-Strahlung mit einer Co-60-Quelle und einer Bestrahlungsdosis von 25kGy erfolgen. Die Alterung kann generell durch Heißluftalterung bei 70°C im Umlufttrockenofen über 7Tage erfolgen.

Wie aus Fig. 3 ersichtlich ist, wurden mit 3 phr Partikel die besten Reißfestigkeiten erzielt, wobei die vernetzten XNBR-Latexfilme durch eine gute Alterungs- und GammaBeständigkeit gekennzeichnet sind.

In weiterer Folge wurde der Einfluss einer Prävulkanisation auf die mechanischen Eigenschaften untersucht. In Fig. 4 sind dazu die entsprechenden Werte dargestellt. Der Partikelanteil betrug jeweils 5 phr. Die Zugfestigkeiten (bestimmt nach ASTM Standard D412-98a, "Standard Test Methods for Vulcanized Rubber and Thermoplastic Elastomers-Tension," Annu. Book ASTM Stand. 09.01 (2002)) sind auf der Ordinate in MPa aufgetragen. Die Balken innerhalb einer Vierergruppe stehen von links nach rechts für nicht sterile und nicht gealterte, nicht sterile und gealterte, sterile und nicht gealterte sowie sterile und gealterte Proben. Auf der Abszisse sind die Vorvernetzungszeiten in Minuten angegeben.

Analog zur Vernetzung mit wasserlöslichen polymeren Epoxidvernetzungsmitteln wird keine Verbesserung der mechanischen Eigenschaften durch eine thermische Vorvernetzung (1-3h bei Temperaturen im Bereich von 50-60°C) beobachtet.

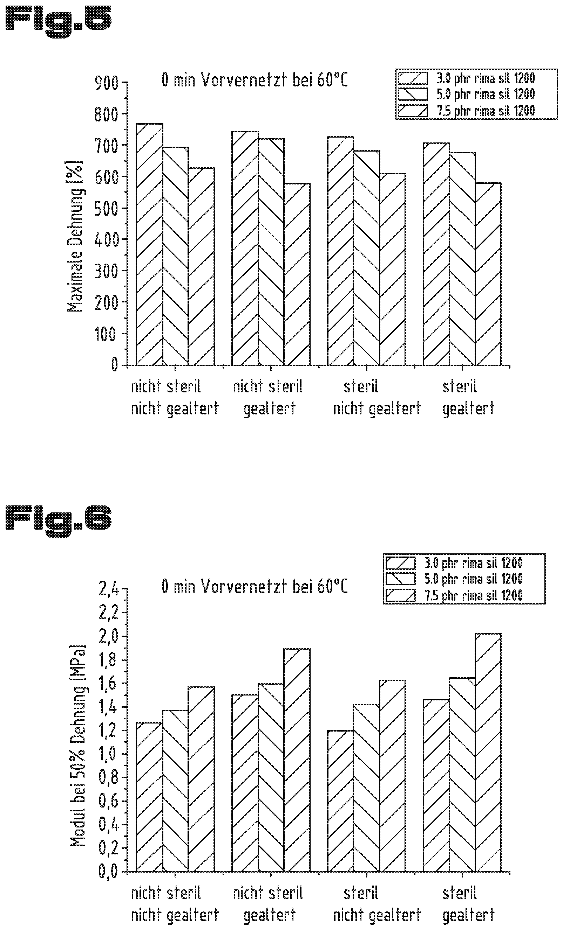

In Fig. 5 sind die Ergebnisse der Dehnungsmessungen und in Fig. 6 die Ergebnisse der Modulmessungen dargestellt. Die entsprechenden Werte können jeweils auf der Ordinate abgelesen werden. Die Abszissenaufteilung entspricht jener der Fig. 3.

Bei Vernetzerkonzentrationen im Bereich von 3 bis 5 phr liegen die Bruchdehnungen durchgängig über 700% und die entsprechenden Spannungswerte bei 50% Dehnung im Bereich von 1,2 bis 1,6 MPa.

Es wurden auch Versuche unternommen, bei denen die Silankomponente der Oberflächenfunktionalisierung direkt und ohne Partikel in die Latexmischung eingebracht wurden. Dabei konnten nur geringere Vernetzungsgrade bzw. schlechtere mechanische Eigenschaften gemessen werden. Dies wurde mit Vergleichen mit CoatOSil MP200 gezeigt, das als organische Hülle auf den Rima Sil Partikeln aufgebracht worden ist. Die Ergebnisse der Zugprüfung zeigen, dass die Reißfestigkeiten unabhängig von der Silankonzentration unter 20 MPa liegen. Eine thermische Vorvernetzung der Latexmischungen bei 60°C führt zu einer zusätzlichen Verringerung der Festigkeiten auf unter 10 MPa. Dies ist möglicherweise auf eine Kondensation der Silane zu Oligokieselsäuren zurückzuführen und zeigt, dass die Partikel eine besondere Funktion für hohe mechanische Festigkeiten des Elastomerfilms haben.

Um die Anwendbarkeit dieser des Verfahrens auch mit weiteren Füllstofftypen zu zeigen, wurden Zeolithe mit funktionellen Silanen modifiziert. Hierbei haben ausführliche Untersuchungen gezeigt, dass die Funktionalisierung von Zeolithen und Kieselsäuren durch die Kondensationsreaktion mit Trialkoxysilanen gelingt. Es wurden Modifikationsgrade bis 5 Gew.-% für Zeolithe und 6 Gew.-% für Silikatpartikel (bezogen auf den anorganischen Träger) realisiert (nachgewiesen mittels Thermogravimetrie). Auch hier konnte beobachtet werden, dass die Epoxygruppen im Zuge der Modifizierung zumindest teilweise hydrolisiert werden und dadurch die verminderte Reaktivität auftritt (dies wurde mittels spektroskopischer Methoden und Korrelation mit den entsprechenden mechanischen Eigenschaften gezeigt). Aus diesem Grund ist es von Vorteil, wie voranstehen beschrieben, beschichtete Partikel einzusetzen, d.h. Partikel, bei denen die erste Lage zwar chemisch an der Partikeloberfläche gebunden und hydrolysiert ist, sich aber bei Silanüberschuss ein Multischichtsystem ausbildet, das nicht hydrolisierte Ankergruppen aufweist. Auf diesem Prinzip beruht auch die voranstehend beschriebene verbesserte Vernetzung mit Rima Sil 1200 Partikeln, da die Ca-Silikate mit einem deutlichen Überschuss an Silan funktionalisiert worden sind und die Organosilanhülle bereits als Beschichtung gesehen werden kann.

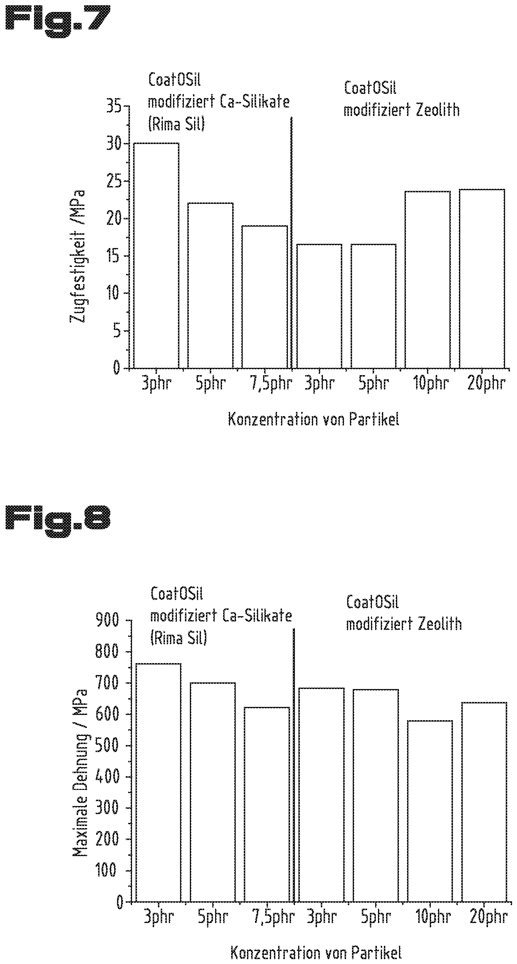

Basierend auf diesen Untersuchungen wurden Zeolithe und Kieselsäuren mit einem Überschuss an Silan modifiziert und als Vernetzungsmittel für die thermische Vernetzung von XNBR-Latex eingesetzt. Zur Modifizierung wurden im ersten Schritt Zeolithsuspensionen (Zeolith 1 bis 4) in Ethanol mit einer Konzentration von 100 %(w/v) hergestellt und anschließend 50 %(w/v) 3-Glycidoxypropyltrimethoxysilan bzw. 50 %(w/v) CoatOSil MP200 zugegeben. Die Suspensionen werden für eine Stunde bei Raumtemperatur mit einem Magnetrührer gemischt und im Anschluss die Partikel für 2 h bei 120°C getrocknet (entfernen des Lösungsmittels). Die Ergebnisse sind in den Fig. 7 bis 8 dargestellt.

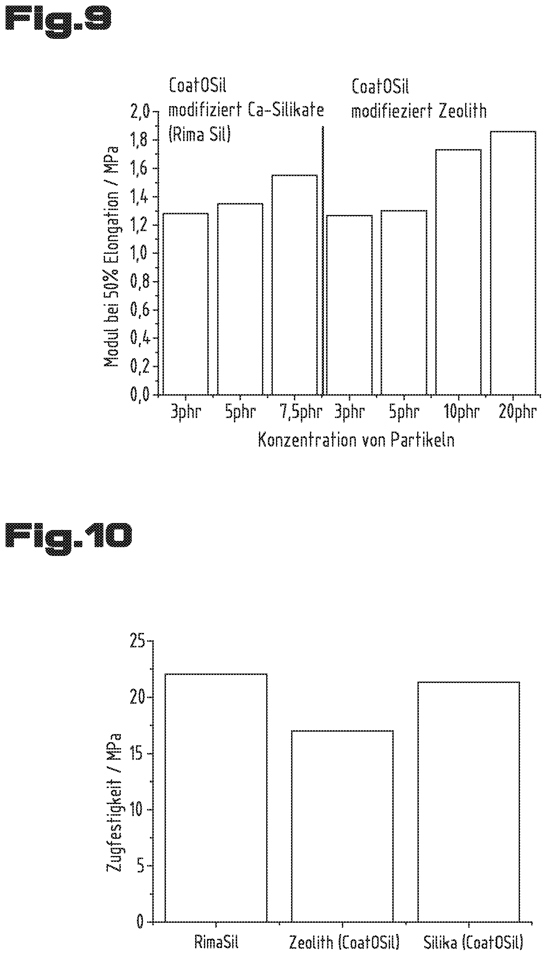

In diesen Fig. sind jeweils mit CoatOSil modifierte Ca-Silikate (links neben der Vertikallinie) den entsprechend modifizierten Zeolithen (rechts neben der Vertikallinie)gegenübergestellt. In Fig. 7 ist auf der Ordinate die Reißfestigkeit in MPa, in Fig. 8 die Dehnung in MPa und in Fig. 9 das Modul bei 50 % Dehnung aufgetragen. Auf den Abszissen sind die Mengenanteile an Partikel im Latex in phr aufgetragen.

Es wurde im Zuge der Arbeiten auch der Einfluss der Partikelgröße auf die mechanischen Eigenschaften untersucht. Bei den verwendeten beschichteten Zeolithpartikeln konnte kein signifikanter Einfluss der Partikelgröße beobachtet werden. Die Reißfestigkeiten von vernetzten XNBR-Latexfilmen bei Zugabe von 10 phr Zeolithpartikel, modifiziert mit CoatOSil MP200 (keine Vorvernetzung; thermische Vernetzung im Zuge der Trocknung bei 100°C für 15min) lagen durchweg zwischen 22 MPa und 25 MPa.

Mit amorphen Kieselsäuren und Ca-Silikaten (Rima Sil) können bei geringeren Vernetzerkonzentrationen (5phr) höhere mechanische Festigkeiten erzielt werden. Die Reißfestigkeiten von vernetzten XNBR-Latexfilmen bei Zugabe von 5phr Partikel modifiziert mit CoatOSil MP200 (keine Vorvernetzung; thermische Vernetzung im Zuge der Trocknung bei 100°C für 15min) lagen um ca. 30 % höher als jene von den untersuchten Zeolithen, woraus auf den Einfluss des Partikelmaterials geschlossen werden kann.

Analog zur Modifizierung von Zeolithen wurden auch Silikapartikel (Silikat 1, Silikat 2, Kieselsäure mit der Bezeichnung KS 400 von der Firma Grace) mittels CoatOSil MP200 entsprechend der voranstehenden Modifizierungsvorschrift beschichtet. Die mechanischen Eigenschaften der enstprechenden XNBR-Latexfilme (keine Vorvernetzung; thermische Vernetzung während der Trocknung bei 100°C) sind mit den Ergebnissen der Rima Sil Partikeln vergleichbar. Bei 5phr können bei Einsatz der modifizierten Zeolithpartikel etwas geringere Reißfestigkeiten beobachtet werden (Fig. 10; Ordinate: Reißfestigkeit in MPa, Abszisse von links nach rechts: Rima Sil, Zeolith, Silica), was mit hoher Wahrscheinlichkeit auf die poröse Füllstoffstruktur (und die damit verbundenen verlängerten Migrationswege des nicht kovalent gebundenen Epoxidvernetzungsmittels) zurückzuführen ist.

In einem weiteren Experiment wurde die thermische Vernetzung von XNBR-Latex mit kommerziell verfügbaren epoxy-funktionellen Kieselerden von Hoffmann Minerals durchgeführt. Der Modifikationsgrad dieser Partikel (Aktisil EM) ist wesentlich geringer, als bei den Rima Sil 1200 Partikeln, während sich die Partikel größen in einem ähnlichen Bereich bewegen, wie dies aus nachstehender Tabelle 2 ersichtlich ist.

**Tabelle 2:**

| | RimasilSil 1200 | Aktisil EM |
|---|---|---|
| Hersteller | Grolman | Hoffmann Minerals |
| d₅₀ [µm] | 2 | 2,2 |
| anorganischer Träger | Ca-Silikat (CaSiO₃) | Kieselerde |
| Organische Hülle | "hoch" (∼20-%) | "niedrig" (∼2%) |

Die mechanischen Festigkeiten der vernetzten XNBR-Latexfilme liegen im Bereich der unvernetzten Referenz (unvernetzter getauchter und getrockneter XNBR-Latexfilm ohne Zugabe von jeglichen Vernetzungschemikalien und Füllstoffen, pH-Wert von 10 der Latexmischung wurde eingestellt) und lassen auf eine unzureichende Vernetzung schließen. Die Ergebnisse bestätigen den Einfluss des Modifikationsgrades der funktionalisierten Partikeln auf die Vernetzungsdichte und damit verbunden die mechanischen Eigenschaften der vernetzten XNBR-Latexfilme. Es wurde gefunden, dass ein Modifikationsgrad der Partikel, gemessen mittels thermogravimetrischer Analyse (TGA) von zumindest 2 %, insbesondere zwischen 4 % und 60 %, von Vorteil ist.

Im Folgenden sind die Versuchsergebnisse für die Ausführung des Verfahrens mit mehrfachfunktionellen Monomeren und/oder Polymeren als weiteres Vernetzungsmittel wiedergegeben. Diese sind jedoch nicht Gegenstand der Erfindung, soweit sie die zusätzliche Zugabe des weiteren Vernetzungsmittels neben den modifizierten Partikel betrifft. In Tabelle 3 sind die hierfür verwendeten Edukte zusammengefasst.

**Tabelle 3: verwendete Materialien zur Vernetzung mit Monomeren und/oder Polymeren**

| *Name* | *Funktion* | *Beschreibung* |
|---|---|---|
| Nipol LX556 ZEON Corporation (JPN) | Latex | 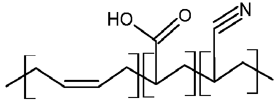 |
| BST8502N | | XNBR |
| PolyLac 582N | | Trockenkautschukgehalt: 45,2% pH-Wert: 8 bis 8.8 |
| SPE, Epoxy-Sorbit CVC Thermo-set Specialities | Polymeres Vernetzungsmittel | 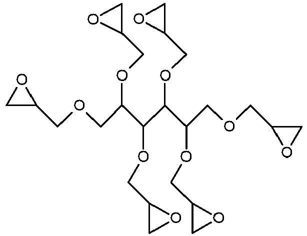 |
| | | Sorbitol polyglycidyl ether (ERISYS GE 60) |
| GE100 Raschig | Polymeres Vernetzungsmittel | 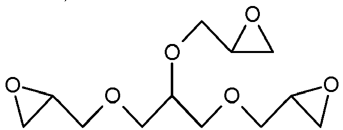 |
| | | Glycerol glycidyl ether |
| DEPEG Sigma-Aldrich (USA) PolyScience (USA) | Polymeres Vernetzungsmittel | 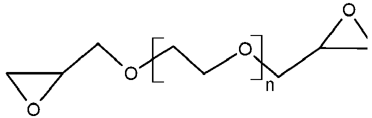 |
| | | Diepoxy-terminiertes Polyethylenglykol |
| | | DEPEG-200 Mn=200 |
| | | DEPEG-500 Mn=500 |
| | | DEPEG-1000 Mn=1000 |

### Herstellung der Latexmischungen, Tauchung und Vernetzung

Das wasserlösliche Vernetzungsmittel wurde in unterschiedlichen Konzentration (0,5 bis 7,5phr) der Latexmischung (pH = 10, ∼25 drc.), die entsprechend voranstehenden Ausführungen modifizierte Partikel enthält, zugegeben. Anschließend wurde die Mischung mit einem Alterungsschutzmittel (2 phr Ralox) versetzt und etwa 15min bei Raumtemperatur gerührt. Anschließend wurden die Filme mittels dem voranstehend beschriebenen Koagulationstauchverfahren hergestellt und die Filme bei 100°C für 15min getrocknet. Es wurde keine Vorvernetzung bzw. Latexreifung benötigt. Die Vernetzung fand während der Trocknung der Filme bei 100°C statt.

Nachfolgende Reaktionen liegen der thermischen Vernetzung mit monomeren und/oder polymeren Epoxidvernetzern zu Grunde. Von Vorteil ist im Vorfeld die Einstellung des pH-Werts der Latexmischung, beispielsweise mit 1 Gew.-%iger KOH auf pH=10, da die Reaktion bei höheren pH-Werten katalysiert wird.

Reaktion eines carboxylierten Elastomers mit einem Epoxid

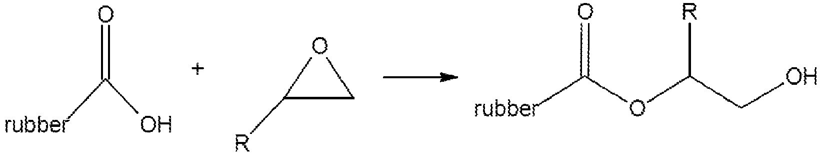

Sauer und basisch katalysierte Ringöffnung von Epoxiden.

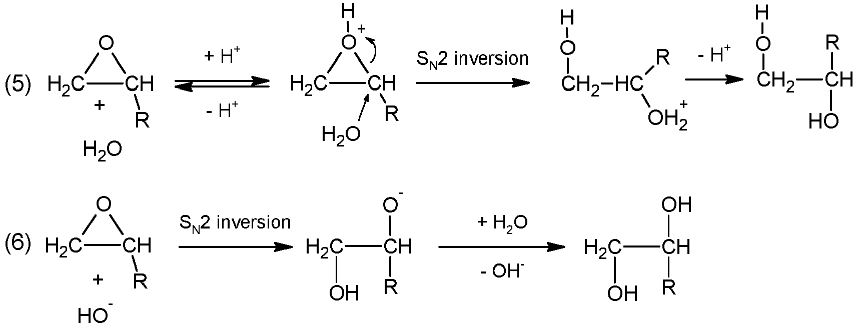

Die erfolgreiche Vernetzung von XNBR-Latex bei Zugabe von ausgewählten wasserlöslichen polymeren Vernetzungsmitteln wurde mittels Gleichgewichtsquellung in Chloroform (Messgrundlage siehe voranstehend) nachgewiesen. Die Vernetzungsdichte steigt hierbei mit zunehmender Vernetzungszeit und Vernetzungsmittelkonzentration wobei die Reaktivität der Vernetzungsmittel von DEPEG-500 < SPE < GE100 zunimmt.

Neben der Gleichgewichtsquellung wurde die Vernetzung von XNBR-Latex bei Zugabe von ausgewählten wasserlöslichen monomeren und polymeren Vernetzungsmitteln auch mittels Zugprüfung nachgewiesen.

Bei Einsatz von DEPEG-500 können ab einer Konzentration von 5phr mechanische Festigkeiten im Bereich von 22 ± 2 MPa beobachtet werden. Bei geringeren Konzentrationen (0,5 bis 3 phr) wird eine geringe Vernetzungsdichte erzielt und die Reißfestigkeiten liegen unter 10 MPa. Eine Erhöhung der Vernetzerkonzentration auf 7,5 phr bringt eine weitere Erhöhung der Festigkeiten von bis zu 35 ± 2 MPa. Bevorzugt wird daher eine Konzentration von 5 phr bis 7,5 phr.

Sehr gute mechanische Festigkeiten und Alterungs- bzw. Gamma-Beständigkeiten wurden auch mit DEPEG-200 in einem Konzentrationsbereich zwischen 3 phr und 7,5 phr beobachtet ((nicht steril/ nicht gealtert: 26 MPa - 40 MPa; nicht steril/gealtert: 37 MPa -26 MPa; steril/nicht gealtert: 28 MPa -24MPa; steril/ gealtert: 25 MPa -35MPa).

Da ähnliche Ergebnisse auch mit anderen mehrfachfunktionellen monomeren und polymeren Vernetzungsmitteln erzielt wurden, wird generell eine Konzentration von 1 phr bis 7,5 phr mehrfachfunktionelle monomere und/oder polymere Vernetzungsmittel im Latex bevorzugt.

Weiter wird eine ausgezeichnete Heißluftalterungs- (7 Tage Lagerung bei 70°C) und GammaBeständigkeit (25 kGy) beobachtet.

Zusätzlich liegt der Spannungswert bei 50% Dehnung auch bei hohen Reißfestigkeiten im Bereich von 1,2 bis 1,4 MPa und wird insbesondere bei Einsatz von 5 phr Vernetzungsmittel auch nach Heißluftalterung und Gammasterilisation kaum erhöht. Dies ist vor allem für die Herstellung von Operationshandschuhen von Vorteil, da ein geringer Spannungswert bei 50 % Dehnung ein Kriterium für einen angenehmen Tragekomfort darstellt.

Analog zur Vernetzung mit DEPEG-500 wurden auch bei Einsatz von SPE (Epoxy-Sorbit) sehr gute mechanische Eigenschaften (auch nach Gamma-Sterilisation) bei höheren Konzentrationen (7,5 phr) nachgewiesen. Bei einer Konzentration von 5 phr SPE wurden Werte für die mechanischen Eigenschaften zwischen 12 MPa und 32 MPa gemessen (nicht steril/ nicht gealtert: 30 MPa - 32 MPa; nicht steril/gealtert: 12 MPa -14 MPa; steril/nicht gealtert: 30 MPa -32MPa; steril/ gealtert: 13 MPa -15MPa). Nachteilig erweist sich jedoch eine geringere Alterungsbeständigkeit der vernetzten XNBR-Latexfilme. Nach einer Lagerung bei 70°C für 7 Tage sinken die Festigkeiten von 30 ± 2 MPa auf unter 15 MPa.

Bei Verwendung von SPE als wasserlösliches hochmolekulares Vernetzungsmittel wird zusätzlich eine ausgeprägte Erhöhung des Spannungswertes bei 50 % Dehnung beobachtet, was nachteilig für den Tragekomfort des Elastomerhandschuhs ist. Bei 7,5 phr SPE werden Werte im Bereich von 1,6 bis 1,8 MPa erhalten.

Bei Einsatz von GE100 als Vernetzungsmittel werden bereits bei geringen Konzentrationen (1 und 3phr) sehr gute mechanische Festigkeiten erzielt, die im Bereich von 20 bis 27 MPa liegen. Mit höheren Vernetzungsmittelkonzentrationen (7,5 phr) wird eine weitere Erhöhung der Reißfestigkeiten beobachtet (37 ± 2MPa). Bei einer Konzentration von 5 phr werden Werte zwischen 22 MPa und 40 MPa erhalten (nicht steril/ nicht gealtert: 35 MPa - 40 MPa; nicht steril/gealtert: 32 MPa -35 MPa; steril/nicht gealtert: 36 MPa -38MPa; steril/ gealtert: 22 MPa -23MPa). Die vernetzten XNBR-Latexfilme zeichnen sich durch eine sehr gute GammaBeständigkeit aus. Als nachteilig erweist sich die geringere Alterungsbeständigkeit, die vor allem bei geringeren Vernetzungsmittelkonzentrationen zu einer Verringerung der Reißfestigkeiten führt.

Zusammenfassend kann aus den Ergebnissen geschlossen werden, dass hohe Reißfestigkeiten (30 ± 2MPa) und Gamma-Beständigkeiten (nach Gammasterilisation: 30 ± 2MPa) mit allen drei untersuchten Vernetzungsmitteln erzielt worden sind. Hinsichtlich Beständigkeit gegenüber einer Heißluftalterung oder einem geringen Modul bei 50 % Dehnung weist DEPEG-500 klare Vorteile gegenüber GE-100 und SPE auf.

Basierend auf diesen Ergebnissen wurde in weiteren Untersuchungen der Modulwert der vernetzten XNBR-Latexfilme durch das Molekulargewicht des epoxid-terminierten Polyethylenglykolderivats (DEPEG) gezielt eingestellt. Mit niedrigerem Molekulargewicht wird einerseits eine sehr hohe Festigkeit (bis zu 40MPa) erreicht, während der Modul ansteigt. Die ist vor allem für die Herstellung von Untersuchungshandschuhen interessant, wo hohe Festigkeiten im Vordergrund stehen und der Modul (auf Grund der Schichtdicke) nur eine untergeordnete Rolle spielt. Bei XNBR-Filmen, die mit DEPEG-500 (mittleres Molekulargewicht) vernetzt worden sind, erhält man zwar etwas geringere Festigkeiten, aber die Modulwerte sind wesentlich geringer. Diese Variante eignet sich mehr für die Herstellung von Operationshandschuhen, wo das Hauptaugenmerk auf einen niedrigen Modul liegt.

Liegt das Molekulargewicht des Vernetzungsmittels jedoch im Bereich von 1.000 g/mol, kann zwar der 50 % Modulwert unter 1MPa gebracht werden, aber die entsprechenden Reißfestigkeiten liegen auch unter 15MPa. Die Ergebnisse zeigen daher, dass über die Kettenlänge des Vernetzungsmittels eine Balance zwischen Reißfestigkeit und Modul eingestellt werden kann. Es werden daher die voranstehend angeführten Kettenlängen der polymeren Vernetzungsmittel bevorzugt.

In weiteren Untersuchungen wurde PolyLac 582N als weitere alternative Latextype bei unterschiedlichen pH-Werten mit 5 phr DEPEG-200 vernetzt. Die Ergebnisse zeigen deutlich, dass eine erfolgreiche Vernetzung von PolyLac 582N gelingt

Weiter wurden Vernetzungen mit einer Mischung bestehend aus epoxidmodifzierten Partikeln und hochmolekularen Epoxidvernetzern durchgeführt. Dazu wurden epoxy-funktionalisierte Partikel (RimaSil 1200) in unterschiedlichen Konzentrationen (1,5 phr bis 5 phr) in deionisiertem Wasser mit einem ULTRA-TURRAX (10 Minuten bei Raumtemperatur) vordispergiert und im Anschluss der Latexmischung (pH = 10,2 ; ∼25 drc.) zugegeben. Anschließend wurde ein wasserlösliches hochmolekulares, mehrfach funktionelles Epoxid (Diepoxy-terminiertes Polyethylenglykol, DEPEG-200) in unterschiedlicher Konzentration (1,5 bis 5 phr) zugegeben und die Mischung mit einem Alterungsschutzmittel (2 phr Ionol LC) versetzt. Die Mischungen wurden für etwa 15min bei Raumtemperatur gerührt. Anschließend wurden die Filme mittels Koagulationstauchverfahren (siehe voranstehende Ausführungen dazu) hergestellt, wobei die Latexmischung während des Tauchprozesses leicht mit Hilfe eines Magnetrührers gerührt wurde, um eine Sedimentation der Partikel zu unterbinden. Die Filme werden bei 100 °C für 15 Minuten getrocknet. Es wurde keine Vorvernetzung bzw. Latexreifung benötigt, da die Vernetzung während der Trocknung der Filme bei 100 °C stattfand. Von Vorteil ist auch hier die Einstellung des pH-Werts der Latexmischung mit 1 Gew.-%iger KOH auf pH=10,2, da die Reaktion bei höheren pH-Werten katalysiert wird.

Von den hergestellten XNBR-Latexfilme (nicht steril, nicht gealtert) betrugen die Reißfestigkeiten ca. 31 MPa für 1,5 phr DEPEG-200 und 1,5 phr Rima Sil 1200, ca. 28 MPa für 2,5 phr DEPEG-200 und 2,5 phr Rima Sil 1200, ca. 33,5 MPa für 5 phr DEPEG-200 und 2,5 phr Rima Sil 1200, ca. 25 MPa für 3,75 phr DEPEG-200 und 3,75 phr Rima Sil 1200 und ca. 30 MPa für 2,5 phr DEPEG-200 und 5 phr Rima Sil 1200.

Die Ergebnisse der Zugprüfung zeigen, dass auch Vernetzungsmittelmischungen bestehend aus einem funktionellen Füllstoff und einem hochmolekularen, mehrfach funktionellen Epoxid zu sehr guten mechanischen Festigkeiten führen. Vor allem bei niedrigen Vernetzungsmittelkonzentrationen führt die Kombination der ausgewählten Vernetzungsmittel zu hohen Reißfestigkeiten.

Der Modul bei 50% Dehnung dieser Latexfilme betrug ca. 1,35 MPa für 1,5 phr DEPEG-200 und 1,5 phr Rima Sil 1200, ca. 1,45 MPa für 2,5 phr DEPEG-200 und 2,5 phr Rima Sil 1200, ca. 1,4 MPa für 5 phr DEPEG-200 und 2,5 phr Rima Sil 1200, ca. 1,35 MPa für 3,75 phr DEPEG-200 und 3,75 phr Rima Sil 1200 und ca. 1,6 MPa für 2,5 phr DEPEG-200 und 5 phr Rima Sil 1200.

Die Dehnung dieser Latexfilme betrug ca. 700 % für 1,5 phr DEPEG-200 und 1,5 phr Rima Sil 1200, ca. 650 % für 2,5 phr DEPEG-200 und 2,5 phr Rima Sil 1200, ca. 660 % für 5 phr DEPEG-200 und 2,5 phr Rima Sil 1200, ca. 620 % für 3,75 phr DEPEG-200 und 3,75 phr Rima Sil 1200 und ca. 620 % für 2,5 phr DEPEG-200 und 5 phr Rima Sil 1200.

In weiteren Versuchen wurden die getrennte Zugabe von Trialkoxysilan und Zeolith (nicht modifiziert) untersucht. Dazu wurden 5 phr unmodifizierte Zeolithpartikel (mono inzeo 15/5) in deionisiertem Wasser mit einem ULTRA-TURRAX (10 Minuten bei Raumtemperatur) vordispergiert und im Anschluss der Latexmischung (pH = 10,2; ∼25 drc.) zugegeben (Einstellung des pH-Werts der Latexmischung mit 1 Gew.-%iger KOH auf pH=10,2). In einem zweiten Schritt wurde das Trialkoxysilan CoatOSil MP 200 mit einer Konzentration von 2,5 phr (Anmerkung: Konzentration des Silans wurde mit der Konzentration der modifizierten Partikel abgestimmt, sodass die Konzentrationen des Silans in der Gesamtmischung in beiden Fällen - getrennte Zugabe und Zugabe von modifizierten Partikeln - gleich hoch ist) der Mischung zugegeben. Anschließend wurde die Mischung mit einem Alterungsschutzmittel (0,5 phr Ionol LC) versetzt und für etwa 15 Minuten bei Raumtemperatur gerührt. Die Filme wurden mittels Koagulationstauchverfahren (siehe voranstehend) hergestellt, wobei die Latexmischung während des Tauchprozesses leicht mit Hilfe eines Magnetrührers gerührt wird, um eine Sedimentation der Partikel zu unterbinden. Die Filme werden bei 100 °C für 15 Minuten getrocknet.

Die Reißfestigkeiten der XNBR-Latexfilme (nicht steril, nicht gealtert) betrugen ca. 11 MPa für 5 phr unmodifizierte Zeolithpartikel, ca. 14 MPa für die getrennte Zugabe von 5 phr unmodifizierten Zeolithpartikeln und 2,5 phr CoatOSil MP 200 und ca. 17,5 für die Zugabe von 5phr modifizierten Zeolithpartikeln (modifiziert mit CoatOSil MP 200).

Aus den Ergebnissen der Zugprüfung kann geschlossen werden, dass die Zugabe von CoatOSil MP 200 zu einer Erhöhung der Reißfestigkeit führt (von 11 auf 14 MPa). Die mechanischen Eigenschaften der XNBR-Latexfilme, die mit modifizierten Partikeln vernetzt worden sind (17,5 MPa), konnten jedoch durch die getrennte Zugabe nicht erreicht werden. Dies zeigt wiederholt den Vorteil der modifizierten Partikel in Hinblick auf eine effizientere Vernetzung und in Hinblick auf gute mechanische Eigenschaften.

Um die Verwendbarkeit von organischen Partikeln zu untermauern, wurden Vernetzungen mit modifizierten Füllstoffen mit organischem Trägermaterialien durchgeführt. Die Herstellung der vernetzten Latexmischungen erfolgte wie voranstehend beschrieben. Für die Vernetzung wurden modifizierte Füllstoffe von der Firma Grolman Chemikalien Handelsgesellschaft mbH eingesetzt, die analog zu den Rima Sil 1200 Partikeln mit CoatOSil MP 200 modifiziert worden sind. Anstelle des Ca-Silikatträgers wurde von Grolman ein vernetztes Silikonharnstoffderivat eingesetzt (Rima Process).

Die Reißfestigkeiten dieser XNBR-Latexfilme wurden für nicht sterile und nicht gealterte Proben mit ca. 27,5 MPa für 3 phr Rima Process, mit ca. 22,5 für 5,0 phr Rima Process und mit ca. 20 MPa für 7,5 phr Rima Process, für nicht sterile und gealterte Proben mit ca. 34 MPa für 3 phr Rima Process, mit ca. 33 für 5,0 phr Rima Process und mit ca. 22,5 MPa für 7,5 phr Rima Process, für sterile und nicht gealterte Proben mit ca. 22,5 MPa für 3 phr Rima Process, mit ca. 23 für 5,0 phr Rima Process und mit ca. 18 MPa für 7,5 phr Rima Process und für sterile und gealterte Proben mit ca. 30 MPa für 3 phr Rima Process, mit ca. 35 für 5,0 phr Rima Process und mit ca. 29 MPa für 7,5 phr Rima Process bestimmt.

Die Ergebnisse der Zugprüfung lassen den Schluss zu, dass eine erfolgreiche Vernetzung auch mit einem organischen Trägermaterial gelingt - d.h. auch ohne eine ionische Vernetzung können durch die kovalente Vernetzung der Carboxylatgruppen hohe mechanische Festigkeiten erzielt werden.

Die Bruchdehnung dieser Latexfilme betrug für nicht sterile und nicht gealterte Proben ca. 740 % für 3 phr Rima Process, ca. 700 % für 5,0 phr Rima Process und ca. 600 % für 7,5 phr Rima Process, für nicht sterile und gealterte Proben ca. 720 % für 3 phr Rima Process, ca. 690 % für 5,0 phr Rima Process und ca. 570 % für 7,5 phr Rima Process, für sterile und nicht gealterte Proben ca. 710 % für 3 phr Rima Process, ca. 650 % für 5 phr Rima Process und ca. 570 % für 7,5 phr Rima Process und für sterile und gealterte Proben ca. 660 % für 3 phr Rima Process, ca. 650 % für 5,0 phr Rima Process und ca. 560 % für 7,5 phr Rima Process.

Der Modul bei 50% Dehnung dieser Latexfilme betrug für nicht sterile und nicht gealterte Proben ca. 1,3 MPa für 3 phr Rima Process, ca. 1,45 MPa für 5,0 phr Rima Process und ca. 1,8 MPa für 7,5 phr Rima Process, für nicht sterile und gealterte Proben ca. 1,5 MPa für 3 phr Rima Process, ca. 1,7 MPa für 5,0 phr Rima Process und ca. 2 MPa für 7,5 phr Rima Process, für sterile und nicht gealterte Proben ca. 1,25 MPa für 3 phr Rima Process, ca. 1,45 MPa für 5 phr Rima Process und ca. 1,82 MPa für 7,5 phr Rima Process und für sterile und gealterte Proben ca. 1,52 MPa für 3 phr Rima Process, ca. 1,8 MPa für 5,0 phr Rima Process und ca. 2,2 MPa für 7,5 phr Rima Process.

In den nachfolgenden Ausführungsbeispielen soll gezeigt werden, dass die thermische Vernetzung von XNBR-Latexfilmen nicht nur mit polaren, wasserlöslichen Epoxidvernetzungsmitteln sondern auch mit unpolareren Epoxidderivaten gelingt.

### Beispiel A - Vernetzung mit Bisphenol A Diglycidylether

3 phr Bisphenol A Diglycidylether (Huntsman) werden in 6 phr deionisiertem Wasser mit 0,3 phr Tween 20 emulgiert. Anschließend wird die Emulsion der Latexmischung (pH = 10,2; ∼25 drc.) zugegeben und die Latexmischung für 60 Minuten bei Raumtemperatur gerührt. Die Filme werden analog zur beschriebenen Durchführung hergestellt und die thermische Vernetzung erfolgt im Zuge der Trocknung der Filme im Umlufttrockenschrank.

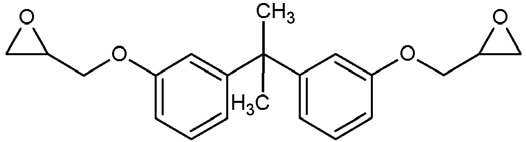

### Bisphenol A Diglycidylether

### Beispiel B - Vernetzung mit einem hydrierten Bisphenol A Diglycidylether

Die Herstellung erfolgt analog zu Beispiel A - nur anstelle des Bisphenol A Diglycidylethers werden 3 phr bzw. 5 phr eines hydrierten Bisphenol A Diglycidylethers (EPALLOY®5000 und EPALLOY®5001 von CVC Thermoset Specialities) eingesetzt.

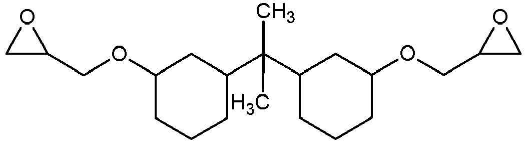

### Hydrierter Bisphenol A Diglycidylether

### Beispiel C - Vernetzung mit einem Hexahydrophtalsäurediglycidylether

Die Herstellung erfolgt analog zu Beispiel A - nur anstelle des Bisphenol A Diglycidylethers wird ein Hexahydrophtalsäurediglycidylether (3 phr und 5 phr EPALLOY®5200 von CVC Thermoset Specialities) eingesetzt

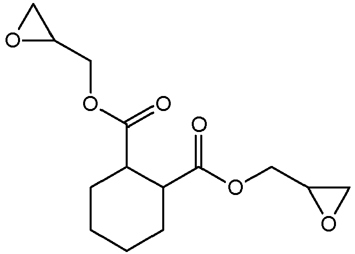

### Hexahydrophtalsäurediglycidylether

### Beispiel D - Vernetzung mit einem 1,4-Cyclohexandimethanoldiglycidylether

Die Herstellung erfolgt analog zu Beispiel A - nur anstelle des Bisphenol A Diglycidylethers wird ein 1,4-Cyclohexandimethanoldiglycidylether (3 phr und 5 phr ERISYS™ GE 22 von CVC Thermoset Specialities) eingesetzt.

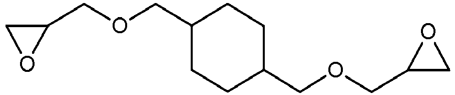

### 1,4-Cyclohexandimethanoldiglycidylether

Die gemessenen mechanischen Eigenschaften der gemäß den Beispielen A-D vernetzten XNBR-Latices sind in Tabelle 4 zusammengefasst.

**Tabelle 4 - Mechanische Eigenschaften von thermisch vernetzten XNBR-Latexfilmen bei Einsatz von unterschiedlichen Epoxiden**

| Epoxidvernetzer | Konzentration Vernetzer [phr] | Reißfestigkeit [MPa] | Spannnung [%] | Spannung bei 50% Dehnung [MPa] |
|---|---|---|---|---|
| Bisphenol A Diglycidylether | 3 | 42,2 | 700 | 1,58 |
| EPALLOY®5000 | 3 | 39,3 | 700 | 1,53 |
| EPALLOY®5000 | 5 | 39,9 | 670 | 1,53 |
| EPALLOY®5001 | 3 | 36,9 | 680 | 1,62 |
| EPALLOY®5001 | 5 | 38,4 | 670 | 1,59 |
| EPALLOY®5200 | 3 | 36,8 | 690 | 1,59 |
| EPALLOY®5200 | 5 | 38,4 | 690 | 1,46 |
| ERISYS™ GE22 | 3 | 34,0 | 680 | 1,60 |
| ERISYS™ GE22 | 5 | 34,9 | 670 | 1,48 |

Die Ausführungsbeispiele beschreiben mögliche Ausführungsvarianten des Verfahrens.

## Patentansprüche

1. Verfahren zum Herstellen eines Prophylaxeartikels, insbesondere eines Handschuhs, aus einem (carboxylierten) Dienkautschuk, nach dem auf eine Form zumindest eine Schicht aus einem (carboxylierten) Dienlatex aufgebracht wird, und der (carboxylierte) Dienlatex mit einem Vernetzungsmittel vernetzt wird, **dadurch gekennzeichnet, dass** das Vernetzungsmittel auf anorganischen und/oder organischen Partikeln unter Bildung von modifizierten Partikel immobilisiert wird und die modifizierten Partikel dem (carboxylierten) Dienlatex zugesetzt werden und dass als Vernetzungsmittel ausschließlich die modifizierten Partikel verwendet werden.

2. Verfahren nach Anspruch 1, **dadurch gekennzeichnet, dass** als anorganische Partikel silikatbasierende Partikel eingesetzt werden.

3. Verfahren nach Anspruch 2, **dadurch gekennzeichnet, dass** die silikatbasierenden Partikel ausgewählt werden aus einer Gruppe bestehend aus Silikaten mit mehrwertigen Kationen, Zeolithen, SiO₂ sowie Mischungen daraus.

4. Verfahren nach Anspruch 3, **dadurch gekennzeichnet, dass** für die Zeolithpartikel ein natürlicher Zeolith verwendet wird.

5. Verfahren nach Anspruch 4, **dadurch gekennzeichnet, dass** als natürlicher Zeolith ein Zeolith verwendet wird, der ausgewählt ist aus einer Gruppe bestehend aus Klinoptilolith, Chabasit, Phillipsit, Analcim sowie Mischungen hiervon.

6. Verfahren nach einem der Ansprüche 1 bis 5, **dadurch gekennzeichnet, dass** die Partikel mit einem Überschuss an Vernetzungsmittel zur Ausbildung eine Mehrschichtstruktur des Vernetzungsmittels auf den Partikeln, modifiziert werden.

7. Verfahren nach einem der Ansprüche 1 bis 6, **dadurch gekennzeichnet, dass** die Vernetzung der (carboxylierten) Dienlatexmoleküle thermisch durchgeführt wird.

8. Verfahren nach einem der Ansprüche 1 bis 7, **dadurch gekennzeichnet, dass** der pH-Wert des (carboxylierten) Dienlatex auf einen Wert von größer/gleich 9 eingestellt wird.

9. Verfahren nach einem der Ansprüche 1 bis 8, **dadurch gekennzeichnet, dass** das Vernetzungsmittel ausgewählt wird aus einer Gruppe bestehend aus mehrfachfunktionellen Epoxiden, mehrfachfunktionellen Silanen, mehrfachfunktionellen Siloxanen, mehrfachfunktionellen Thiolen, sowie Mischungen daraus.

10. Prophylaxeartikel, insbesondere Handschuh, umfassend eine Schicht aus einem (carboxylierten) Dienelastomer, wobei die (carboxylierten) Dienelastomermolekülketten des (carboxylierten) Dienelastomers kovalent über organische Moleküle und ionisch über Metallkationen vernetzt sind, **dadurch gekennzeichnet, dass** die Metallkationen ausschließlich Teil von anorganischen Partikeln sind, und dass die organischen Moleküle ausschließlich auf den anorganischen Partikeln immobilisiert sind.

## Claims

1. A method for the manufacture of a prophylactic article, in particular of a glove, from a (carboxylated) diene rubber, according to which at least one layer of a (carboxylated) diene latex is applied on a former and the (carboxylated) diene latex is cross-linked with a cross-linking agent, **characterized in that** the cross-linking agent is immobilized on inorganic and/or organic particles forming modified particles, and the modified particles are added to the (carboxylated) diene latex and **in that** exclusively the modified particles are used as cross-linking agents.

2. The method according to claim 1, **characterized in that** silicate-based particles are used as inorganic particles.

3. The method according to claim 2, **characterized in that** the silicate-based particles are selected from a group consisting of silicates with multivalent cations, zeolites, SiO2 as well as mixtures thereof.

4. The method according to claim 3, **characterized in that** a natural zeolite is used as the zeolite particles.

5. The method according to claim 4, **characterized in that** a zeolite selected from a group consisting of clinoptilolite, chabasite, phillipsite and analcime as well as mixtures thereof is used as natural zeolite.

6. The method according to one of claims 1 to 5, **characterized in that** the particles are modified with an excess of cross-linking agent for formation of a multi-layer structure of the cross-linking agent on the particles.

7. The method according to one of claims 1 to 6, **characterized in that** the cross-linking of the (carboxylated) diene latex molecules is carried out thermally.

8. The method according to one of claims 1 to 7, **characterized in that** the pH of the (carboxylated) diene latex is adjusted to a value of greater than/equal to 9.

9. The method according to one of claims 1 to 8, **characterized in that** the cross-linking agent is selected from a group consisting of multifunctional epoxides, multifunctional silanes, multifunctional siloxanes, multifunctional thiols, as well as mixtures thereof.

10. A prophylactic article, in particular glove, comprising a layer of a (carboxylated) diene elastomer, wherein the (carboxylated) diene elastomer molecular chains of the (carboxylated) diene elastomer are cross-linked covalently via organic molecules and ionically via metal cations, **characterized in that** the metal cations are exclusively part of inorganic particles, and **in that** the organic molecules are exclusively immobilized on the inorganic particles.

## Revendications

1. Procédé de fabrication d'un article prophylactique, en particulier d'un gant, à partir d'un caoutchouc diène (carboxylé), selon lequel on applique sur une forme au moins une couche d'un latex diénique (carboxylé), et le latex diénique (carboxylé) est réticulé à l'aide d'un agent de réticulation, **caractérisé en ce que** l'agent de réticulation est immobilisé sur des particules inorganiques et/ou organiques avec formation de particules modifiées, et les particules modifiées sont ajoutées au latex diénique (carboxylé), et **en ce qu'**on utilise en tant qu'agent de réticulation exclusivement les particules modifiées.

2. Procédé selon la revendication 1, **caractérisé en ce qu'**on utilise en tant que particules inorganiques des particules à base de silicates.

3. Procédé selon la revendication 2, **caractérisé en ce que** les particules à base de silicates sont choisies dans un groupe consistant en les silicates comportant des cations polyvalents, les zéolites, SiO₂, ainsi que les mélanges de ceux-ci.

4. Procédé selon la revendication 3, **caractérisé en ce qu'**on utilise pour les particules de zéolite une zéolite naturelle.

5. Procédé selon la revendication 4, **caractérisé en ce qu'**on utilise en tant que zéolite naturelle une zéolite qui est choisie dans un groupe consistant en la clonoptilolite, la chabasite, la phillipsite, l'analcime, ainsi que les mélanges de ceux-ci.

6. Procédé selon l'une des revendications 1 à 5, **caractérisé en ce que** les particules sont modifiées à l'aide d'un excès d'agent de réticulation, pour la formation d'une structure multicouche de l'agent de réticulation sur les particules.

7. Procédé selon l'une des revendications 1 à 6, **caractérisé en ce que** la réticulation des molécules de latex diénique (carboxylé) est mise en œuvre par voie thermique.

8. Procédé selon l'une des revendications 1 à 7, **caractérisé en ce que** le pH du latex diénique (carboxylé) est ajusté à une valeur supérieure ou égale à 9.

9. Procédé selon l'une des revendications 1 à 8, **caractérisé en ce que** l'agent de réticulation est choisi dans un groupe consistant en les époxydes multifonctionnels, les silanes multifonctionnels, les siloxanes multifonctionnels, les thiols multifonctionnels, ainsi que les mélanges de ceux-ci.

10. Article prophylactique, en particulier gant, comprenant une couche d'un élastomère diénique (carboxylé), les chaînes moléculaires de l'élastomère diénique (carboxylé) de l'élastomère diénique (carboxylé) étant réticulées par voie covalente par l'intermédiaire de molécules organiques et par voie ionique par l'intermédiaire de cations métalliques, **caractérisé en ce que** les cations métalliques sont exclusivement une partie de particules organiques, et **en ce que** les molécules organiques sont exclusivement immobilisées sur les particules inorganiques.
